(19)

Europäisches
Patentamt

European
Patent Office

Office européen
des brevets

(11) **EP 4 154 977 A1**

(12) **EUROPEAN PATENT APPLICATION**
published in accordance with Art. 153(4) EPC

(43) Date of publication:
**29.03.2023 Bulletin 2023/13**

(21) Application number: **20747445.3**

(22) Date of filing: **09.06.2020**

(51) International Patent Classification (IPC):
**B01L 3/00** $^{(2006.01)}$      **A61B 5/145** $^{(2006.01)}$
**A61B 5/00** $^{(2006.01)}$

(52) Cooperative Patent Classification (CPC):
**B01L 3/502715; A61B 5/14517; A61B 5/4266;**
**A61B 5/6801; B01L 3/502738; B01L 3/502746;**
A61B 5/7275; B01L 2200/0621; B01L 2200/0684;
B01L 2200/10; B01L 2300/0627; B01L 2300/16;
B01L 2400/0406; B01L 2400/0605; B01L 2400/0688

(86) International application number:
**PCT/ES2020/070381**

(87) International publication number:
**WO 2021/250288 (16.12.2021 Gazette 2021/50)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **Onalabs Inno-Hub S.L.**
**08012 Barcelona (ES)**
• **Universitat Politècnica de Catalunya**
**08034 Barcelona (ES)**

(72) Inventors:
• **RABOST GARCÍA, Genis**
**08012 Barcelona (ES)**
• **MUÑOZ PASCUAL, Francisco Javier**
**08012 Barcelona (ES)**
• **CASALS TERRE, Jasmina**
**08222 Terrassa (ES)**
• **FARRÉ LLADÓS, Josep**
**08222 Terrassa (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **MICROFLUIDIC SYSTEM AND METHOD FOR CONTINUOUS MONITORING OF METABOLITES AND/OR PROPERTIES OF BIOFLUIDS**

(57) The present invention refers to a microfluidic system based on passive capillary valves and pumps, that allows a discontinuous and autonomous measurement process for extensive periods of time. The microfluidic system comprises: at least one measuring chamber, at least one inlet for the input of a biofluid, a microfluidic intake channel fluidly communicating the inlet with the measuring chamber, at least one sensor suitable for measuring a parameter of an analyte of a biofluid. A passive fluid pump is fluidly communicated with the measuring chamber, and it is adapted to generate a capillary pressure greater than the biofluid generation pressure. A retention valve is interposed between the measuring chamber and the fluid pump, and the retention passive valve is configured to stop flow of biofluid for a certain period of time, when the measuring chamber if filled with biofluid. The invention provides a robust device, capable of collecting and conveying a biofluid, preferably sweat, for repetitive and electrochemical measurements.

EP 4 154 977 A1

**Description**

**Field and object of the invention**

The present invention refers in general to passive microfluidic systems and methods for continuous monitoring of biofluids, with chronological assurance and repetitive sampling.

An object of the invention is to provide a microfluidic system based on passive capillary valves and pumps, that allows a discontinuous and autonomous measurement process for extensive periods of time.

An additional object of the invention is to provide a robust device, capable of collecting and conveying sweat for repetitive and electrochemical measurements.

The invention preferably belongs to the technical filed of Point-of-Care/Lab-on-a-chip devices, so it can be applied to wearable systems for sweat lactate and glucose monitoring, but it could also be adapted to other biofluids or metabolites.

**Background of the invention**

New technologies that improve the efficiency and reduce the costs associated to the healthcare sector have always been searched. However, the challenges that must be faced in the near future make the development of innovative approaches an obligation to avoid healthcare saturation. The continuous population growth, the aging of society and the revoking of mortal diseases to chronic diseases, are consequences of the progress in medicine, however, they produce an acceleration of the resources needed that healthcare system cannot support. This effect can be exemplified by physician shortage projections which declare that around 150,000 more physicians will be needed to cover the demand in the US by 2025. This problem cannot be solved only by increasing the number of healthcare professionals; a change in healthcare paradigm is needed.

One possible solution could be a telemedicine approach, where patient health status is remotely monitored using integrated chemical sensing on wearable technology. This growing market seeks to reduce costs associated to unnecessary visits while providing new tools and information to clinicians, making the whole follow-up process more efficient. However, the gold standard to extract information about patient condition is still blood analysis which is invasive, causing physical trauma and risk of infection. Besides, there are cases where continuous monitoring via blood is problematic. For example, between 6 and 8 daily blood extractions are done to neonates with congenital heart diseases in ICU which represents one quarter of their total blood for a 10-day stay.

In recent years, sweat has emerged as an alternative, easily accessible, biofluid valid for non-invasive chemosensing. Apart from water (99%), it contains electrolytes (sodium, chloride, potassium ...), metabolites (glucose, lactate, urea... ) and small quantities of sugars, proteins and metal ions. Therefore, sweat presents a wide range of analytes of clinical interest. Among others non-invasive biofluids such as urine, saliva or tears; sweat stands out for the large number of sampling sites, continuous and easy access, stimulation on-demand, comfort for the user and the low degradation of analytes during the process. However, there are several challenges such as irregular sampling, contamination from skin, low volume analysis, mixing of fresh sweat generated with old sweat already present in skin, sample evaporation... All these problems associated to sweat analysis can be solved by innovative technologies, enabling a true leap in wearable technology.

Traditional sweat sampling methods consisted in occlusive or non-occlusive bandages that collected sweat in absorbent materials for posterior analysis. However, occlusive bandages produced skin irritation and yielded small volumes of sweat while non-occlusive gave false concentration measurements as water evaporates through time. Nowadays, commercial devices for sweat sampling use dedicated external instruments to measure a particular analyte of interest.

However, a wearable approach, which can be understood as a PoC (Point of Care) device, is needed. Sweat collection must be combined with a sensor platform whose measured information must be processed and sent remotely by an electronic system (*in situ* detection). Advancements in a variety of fields such as material science, chemical analysis, microsystem design and fabrication and electronics allowed the development of biosensors associated to flexible low-power electronics capable of wireless communication, in other words, bringing sensors directly onto skin. This enabled the publication of different sweat wearable devices in the form of bands, patches or tattoo.

Some sweat wearables started to include microfluidics, that allows fluid manipulation in order to collect and transport it to the detection zone in a controlled manner. Therefore, it can provide a more reliable and reproducible chemical analysis because sensing is carried out in a better controlled environment, avoiding contamination from skin.

The simplest approach is to place the sensors in a channel where sweat will circulate through. One or more inlets lead to an enclosed chamber where an electrochemical sensor is placed. Although providing a more controlled environment to perform the measurement, no control of fluid dynamics is proposed, relying entirely on sweat gland pressure to move the fluid.

Other proposals added a passive pumping system at the end of the microfluidic circuit to establish a constant flow rate inside the device, such as micro-fabricated capillary pumps, porous materials such as paper or using evaporation. This way, with an analyte flow constant and known, the analyte concentration reading can be calculated more accurately.

Some known solutions are based on the use of passive valves. Briefly, they use a collection of passive valves to direct the filling of different chambers for posterior analysis or colorimetric analysis. This way, each chamber collects sweat solely from a certain period of time, assuring the independency between time measurements. However, the large number of sweat chambers limits its applicability with electrochemical measurements because we would need a sensor for each single measurement. That is why they opt to use external analysis or colorimetric transduction.

Passive microfluidics (microfluidics without external pumping systems) has been intensively researched as well as key components of the Point-of-Care systems. A variety of different elements has been described (passive valves and capillary pumps within them) to be able to direct flow in a programmed way. Basically, the purpose of this type of microfluidic systems were focused on the sequential delivery of different fluids and reagents to a chamber in order to replicate in these chips traditional chemical assays and procedures carried out at the lab.

In capillary governed microfluidic devices, filling will be determined by the liquid pressure at the meniscus, the interface liquid-gas. CPVs consist in geometrical or chemical variations that changes liquid pressure and stops the filling process. In the case of geometrical, it is due to an abrupt enlargement of the cross-section area of the channel, which implies a pressure barrier to overcome by the fluid meniscus. Once the liquid pressure exceeds the bursting pressure (BP) of the valve (defined by the dimensions of the channel, the diverging angle and the contact angle), the meniscus bursts and flow is restored. This process is theoretically irreversible because no air-liquid interface is formed again.

Theoretically, the liquid pressure at the meniscus can be defined as the change of interfacial energy on an increase of the filling liquid volume, mathematically expressed in Equation 1 for a rectangular channel where the valve is defined in 2D:

$$\mathrm{BP} = -2\sigma \left[ \frac{\cos\theta_I^*}{b} + \frac{\cos\theta_A}{h} \right], \tag{1}$$

where BP is bursting pressure, $\sigma$ is surface tension of the fluid, b and h are width and height of the channel respectively, $\theta a$ is the advancing contact angle of the fluid-channel and $\theta^*$ is the minimum between $\theta a + \beta$ and 180°, $\beta$ being the diverging angle of the channel.

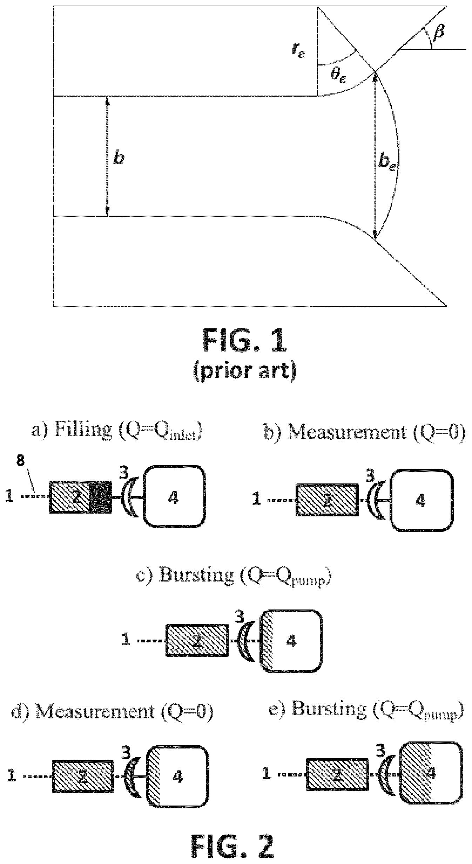

From this basic equation, the main parameters influencing the behaviour of a geometrical capillary passive valve (BP, bursting pressure) as shown in **Figure 1** are: the width (b, inversely proportional to BP), diverging angle ($\beta$, proportional to BP) and contact angle fluid-channel ($\theta a$, proportional to BP too).

In some known solutions, passive fluid pumps used where microfabricated arrays that provide a capillary pressure capable of draining the fluid. The physical principle driving this is the same as in passive valves described before (Equation 2). Basically, a suction pressure is generated by the micrometric structures fabricated, better said, by the spacing between these microstructures (a and b parameters). Therefore, as closer the structures are, fluid paths between the pump will be narrower, and a greater capillary pressure can be provided. Another condition to be met is that the substrate material must be hydrophilic (the cos of the contact angle should be positive). Different structures can be used as pillars, posts, diamonds, channels. Their main influence is not in the capillary pressure provided, instead in minimizing the resistance to flow which results in viscous losses reducing the flow rates achievable.

$$P_c = -\gamma \left( \frac{\cos\alpha_b + \cos\alpha_t}{a} + \frac{\cos\alpha_l + \cos\alpha_r}{b} \right), \tag{2}$$

This kind of pumps, called capillary pumps, are able to provide constant flow rates and drive the flow movement of the whole microfluidic circuit. With this approach, the pump is fabricated during the same procedure of the microchannel fabrication, simplifying steps and materials, but high-resolution fabrication procedures are needed.

Alternatively, inherently porous materials can be used such as paper. For example, cellulose typically has a pore size between 1-10 um combined with an hydrophilic nature. Therefore, the network of microchannels that are artificially created in capillary pumps can be changed for the intricate porous network of this type of materials. This pressure can easily reach several tens of kPa and depends on the structure and moisture content of the porous material.

Although the response will not be as controlled and predictable, they offer a simple solution as passive pumping system. In fact, due to its simplicity and naturally wicking behaviour, they are used as the substrate where fluid travels, integrating sensing systems in their structure. In what we are interested though, is in their integration on microfluidic systems as passive pumping system.

Therefore, there is the need for microfluidic systems for continuous monitoring of biofluids, that features low complexity and reduced dimensions but without losing accuracy or time-resolution and assuring that each measurement is not influenced by previously measured biofluid samples.

## Summary of the invention

The present invention is defined in the attached independent claim, and satisfactorily solves the drawbacks of the prior art, by providing a continuous monitoring system using a single measuring chamber instead of multiple measuring chambers as the case of prior art solutions, such that the complexity and dimensions of the microfluidic system are significantly reduced.

More specifically, an aspect of the invention refers to a microfluidic system for monitoring metabolites and/or properties of biofluids, wherein the system comprises: one measuring chamber, at least one inlet for the input of a biofluid collected from a subject, and a microfluidic intake channel fluidly communicating the inlet with the measuring chamber. Preferably, the inlet is adapted for collecting sweat from the skin of a subject.

In the present disclosure, "continuous monitoring" means the capability to perform multiple single measurements over a period of time of interest with chronological assurance and in a repetitive manner, allowing the obtention of the evolution of a given parameter over that period of time.

The system additionally comprises at least one sensor suitable for measuring a parameter of an analyte of a biofluid, such that the sensor is arranged to measure the parameter of a biofluid contained in the measuring chamber.

In a preferred embodiment, the system comprises a single measuring (or sensing) chamber. This measuring chamber can contain one or multiple sensors for different biomarkers, but there is no need to replicate the sensors at different locations of the microfluidic circuit, as is the case of some prior art proposal.

The system additionally comprises a passive fluid pump, fluidly communicated with the measuring chamber, and adapted to generate a capillary pressure greater than the biofluid generation pressure. In the case of sweat monitoring applications, greater than the sweat gland generation pressures.

In a preferred embodiment, fluid pump is a porous material. Alternatively, the fluid pump is a micro-machined capillary pump capable of forcing fluid circulation by capillary action.

In the present disclosure, "passive" refers to any device which does not use external energy to operate.

Furthermore, the system comprises a retention (or delay) passive valve interposed between the output of the measuring chamber and the fluid pump. The retention passive valve is configured to stop biofluid flow towards the fluid pump for a certain period of time, when the measuring chamber is filled with biofluid.

Preferably, the retention valve is configured to feature a bursting pressure that makes the retention valve to retain the measuring chamber filled with biofluid, and stop biofluid flow for a period within the range 1 minute to 5 hours.

In preferred embodiments, for example for sweat measurements, the previously mentioned period is within the range 1 to 5 minutes.

Therefore, according to the invention the measuring or sensing region ends with a passive valve that temporarily stops the flow, in order to ensure a complete and correct filling of the measuring chamber, and to perform measurements in static conditions (flow velocity is zero). As analyte flow rate is a parameter that affects the response of sensors, the system is configured such that measurements are not affected by this variable, leading to a more reliable analysis.

Preferably, the passive valve is an abrupt or sudden enlargement of the microfluidic intake channel cross-sectional area, as it does not need additional manufacturing/modification steps, but other alternative means such as hydrophobic barriers can also be used.

Alternatively, the retention passive valve is configured as a chemical modification of the surface of the microfluidic intake channel.

Retention times (amount of time the fluid remains stopped until the valve burst) will depend on the specific application of interest (analyte variation over time, total time of the monitoring), but for sweat applications as mentioned above, it should be in the range of 1 to 5 minutes.

The bursting pressure of a passive valve is the parameter typically used to characterize the operation of passive valves, and it has an analytical relationship with geometrical and material parameters. As a rule, the higher the bursting pressure is, the longer delay times.

In a preferred embodiment, the bursting pressure of the retention valve is within the range 2.4 - 6 kPa.

Just after the passive valve, a passive pumping system is integrated so that when fluid bursts through the passive valve, fluid reaches the passive pump by the capillary pressure exerted by the pump, which is greater than the one at the inlet by the biofluid while the inlet is collecting biofluid from a biofluid source.

In a preferred embodiment, the passive fluid pump is adapted to generate a capillary pressure higher than 6 kPa, which is suitable when the system is adapted to monitor sweat metabolites and/or properties, like sweat conductivity, and/or sweat rate, and/or sweat pH, and/or ions for sodium loss monitoring.

It has been found, that this mismatch between the flow rate at which the fluid enters the microfluidic circuit and the flow rate imposed by the passive pump, leads to a disconnection of the fluid flow at the retention valve after several seconds. Therefore, the air-fluid interface needed to restore the operation of the passive valve as retention means, is again created and the fluid is stopped at the passive valve. In addition, the volume of biofluid drained by the passive pump (the already pumped fluid) is no longer in contact with the newly collected bio fluid through the inlet, reducing thereby cross-contamination between different measurements.

An advantage of the invention is that that cycle can be repeated multiple times providing single independent measurements over a prolonged period of time, thus, allowing for an accurate continuous monitoring. Cycle period can be adjusted by selecting dimensions of the microfluidic circuit, passive valve parameters and passive pump properties.

The above-described structure of the system of the invention, overcomes a characteristic limitation of capillary-driven microfluidic elements of the prior art, which is that they only work when there is an air-fluid interface. Typically, capillary passive valves only function when the fluid front arrives and, once surpassed, they behave just as a fluid conduit. Similarly, passive pumps work until they are completely filled. According to the present invention, the system regenerates this air-fluid interface, during its operation in an autonomous, passive way, to restore the function of the surpassed retention valve.

Cycle period should be repetitive if external conditions are kept constant, for instance biofluid generation rate. However, certain environments of application such on-body sweat analysis are highly variable.

Preferably, the system incorporates a cycle detector adapted to detect when the chamber is filled so it can be decided when to perform a measurement, and to monitor changes on cycle frequency that is related to fluid generation rate.

With the above-described system according to the invention, irregular sampling, a particularly undervalued issue about continuous monitoring of sweat analysis, can be addressed.

The cycle detector can be embodied as two electrodes arranged to measure an electric parameter (admittance or impedance) at the microfluidic circuit, that is, the circuit between the inlet and the fluid pump inlet, so as to detect whether there is a biofluid or air in the microfluidic circuit.

In a preferred embodiment, the system includes a secondary microfluidic channel connected to the microfluidic intake channel and connected to the atmosphere, and a stop passive valve interposed at the secondary microfluidic channel, such that the stop valve impede fluid flow out of the secondary microfluidic channel towards the atmosphere.

In particular, the stop valve is configured such that its bursting pressure is greater than the maximum biofluid generation pressure, so that the pressure barrier created would never be surpassed by the fluid pressure. This means, that the bursting pressure of the retention passive valve is lower than the bursting pressure of the stop passive valve.

The characteristics of the following elements and their relationship between them could be better expressed in function of pressure: pressure of fluid generation, bursting pressure barrier for passive valves and pumping pressure for fluid pumps. Below, a detailed description of each parameter is presented.

Sweat Pressure: sweat glands operate by osmotic pressures generated by differences in osmolality between plasma and sweat. Some prior art studies reported a broad range of secretory pressures, from 3 kPa to a maximum of 70 kPa, with mean values around 40 kPa. This maximum pressure of 70 kPa would correspond approximately to around 20 nL/min gland, falling between the range of observed sweat rates.

Other prior art studies found that the secretory pressures lie between 2.4 and 2.9 kPa, with the maximum under 6 kPa. They attributed the differences with the previous study to the pressure drop along the sweat duct (measurement was not carried out at the skin surface, rather in the middle of the sweat duct with no control on the depth) and difference between natural and induced sweating. What is clear is that the last measurements are more related to the secretory pressures that our system will experience.

Bursting Pressure Valves: with reference to the Equation 1 of the BP, and the expected secretory pressures, the geometrical parameters of each passive valve used in the system can be designed accordingly to their function.

The relation between the bursting pressure of the passive valve and the delay time produced because under constant conditions, are directly proportional. Therefore, under the conditions of sweat analysis, the geometrical parameters of the retention valve are designed to be found within the upper range of secretory pressures, allowing to stop the flow but not too strong to restrict completely the flow.

Pumping pressure of fluid pumps: the pumping pressure of the passive pumps considered on the invention is due to the capillary pressure generated by their micrometric features. The flow rate achieved would be a consequence of this capillary pressure and it will depend on the rest of the microfluidic system. That is why, capillary pressure, being an intrinsic parameter of the pumping system that can be clearly compared and related with the rest of pressures involved in the system.

Another aspect of the invention refers to a wearable device for sweat monitoring, that incorporates a system as the one defined above, and wherein the system is configured as a disposable cartridge detachably coupled with the wearable device, and adapted to monitor sweat metabolites and/or sweat properties like sweat conductivity and/or sweat rate, and/or sweat pH, and/or ions.

## Brief description of the drawings

Preferred embodiments of the invention are henceforth described with reference to the accompanying drawings, wherein:

Figure 1.- shows schematically in a top plan view, the structure of a geometrical passive valve according to the prior-art (height constant) where all relevant parameters are noted.

Figure 2.- shows schematically a first embodiment of the microfluidic system of the invention at five sequential stages (a - e) of the measurement process. Q is biofluid flow. In the microfluidic system, dark areas represent air, and striped areas represent liquid flow.

Figure 3.- shows schematically the chronological steps of the system of Figure 2, with a graphical representation of the theoretical fluid flow evolution over time.

Figure 4.- shows schematically the working principle and the different steps of a second embodiment of the microfluidic system, that includes an air vent and a secondary channel.

Figure 5.- shows a practical implementation of the embodiment of Figure 2, wherein Figure A shows the entire system, Figure B is an enlarged detail of the measuring chamber and retention passive valve including three sequential stages (I, II, III) of the process. Figure C shows the measuring chamber of the second embodiment of Figure 4 also including three sequential stages (I, II, III) of the process, and areas for detecting air bubble formation. In the figure, dark areas represent fluid flow.

Figure 6.- shows a wearable device integrating the proposed microfluidic system, wherein Figure A is an exploded view; and Figure B is a schematic representation of the arrangement of layers to implement the system.

Figure 7.- shows a sequece of captions (A - F) of different stages of the cycle carried out in a practial implementation of the microfluidic device.

Figure 8.- shows more captions (A - D) of different stages of the cycle carried out in a practial implementation of the microfluidic device.

## Preferred embodiment of the invention

**Figure 2** shows a first embodiment of the microfluidic system of the invention at five sequential stages (a - e) of the measurement process. The microfluidic system comprises:

- one measuring chamber (2),
- one inlet (1) for the input of a biofluid, for example adapted to collect sweat from the skin of a subject,
- a microfluidic intake channel (8) fluidly communicating the inlet (1) with the measuring chamber (2),
- a passive fluid pump (4) fluidly communicated with the measuring chamber (2), and adapted to generate a capillary pressure greater than the biofluid generation pressure,
- at least one sensor (not shown) suitable for measuring a parameter of an analyte of a biofluid, and arranged to measure the parameter of a biofluid contained in the measuring chamber. Preferably, one sensor is enclosed inside the measuring chamber,
- a retention passive valve (3) interposed between the measuring chamber (2) and the fluid pump (4).

The retention passive valve (3), preferably is of the geometrical type, and it is configured to stop flow of biofluid for a certain period of time (preferably 1 to 5 minutes for sweat applications), when the measuring chamber is filled with biofluid.
The passive pump (4) drains the fluid at a high flow rate, and it only works when the retention valve (3) is surpassed. It could be a naturally porous material (like paper) or a microfabricated pillar-like capillary pump. The relevant characteristic of the passive pump, is that its wicking pressure provides a flow rate greater than the one at the inlet of the microfluidic system. For sweat applications, pump fluid generation rate varies between 1 to 20 nL/min-gland, which can be traduced (accounting 100 glands/cm$^2$ and a collection area of 0.5 cm$^2$) to a 50-1000 nL/min range.

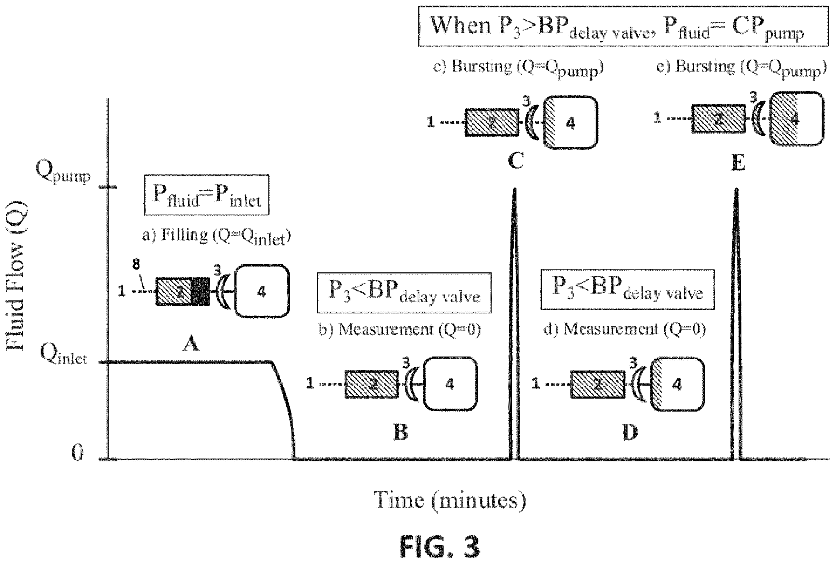

**Figure 3** shows the chronological steps of the operation of the embodiment of **Figure 2** with a graphical representation of the theoretical fluid flow evolution over time, which is as follows:

- Filling, (stage A in Figures 2 & 3). Fluid enters through the inlet (1) and flows through the microfluidic system at the rate of fluid generation.

- Measurement, (stage B in Figures 2 & 3). When the fluid front arrives at the retention passive valve (3), it is stopped for a few minutes, because the dominant pressure governing fluid flow is lower than the bursting pressure (BP) of the retention valve (3). With no fluid flow and with the measuring chamber (2) filled, then measurements are taken while fluid pressure (P3) at the fluid-air interface increases due to the fluid generation pressure at the inlet.

- Bursting, (stage C in Figures 2 & 3). After a certain period of time, the retention passive valve (3) bursts due to the increased fluid pressure, and fluid flows through it (at a high flow rate (note first flow peak in Figure 3), and reaches the passive pump (4) flowing through the pump.

- Next measurement, (stage D in Figures 2 & 3). Then, after a few seconds due to the high flow rate forced by the pump (4), the flow disconnects, that is, the fluid flow is interrupted and an air-fluid interface is regenerated at the passive valve (3), and the fluid is stopped again and measurements in the measuring chamber are taken again.

- Bursting, (Stage E in Figures 2 & 3). After several minutes, bursting occurs again (second flow peak), repeating the cycle described above.

In **Figure 3**, $P_3$ is the fluid pressure at position 3 while $P_{fluid}$ refers to the dominant pressure governing fluid flow, BP is bursting pressure of the retention (or delay) passive valve (3), and CP is capillary pressure of the pump (4).

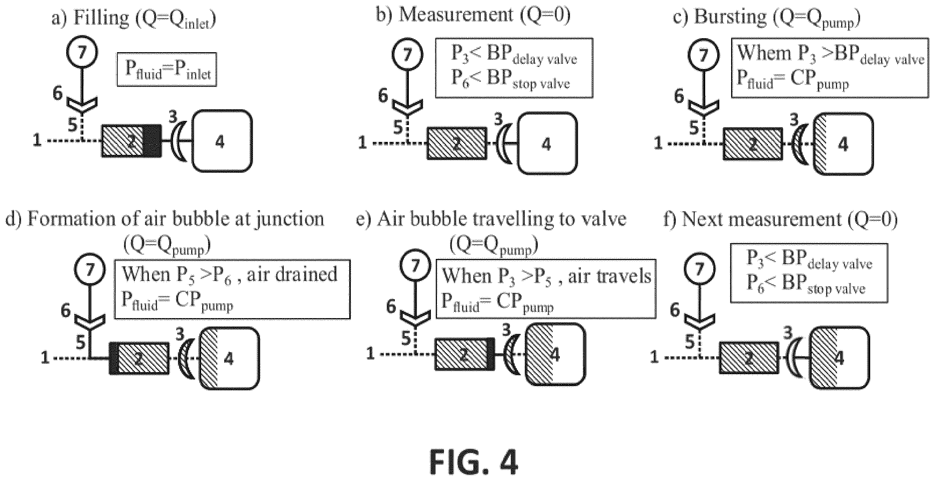

**Figure 4** shows a second embodiment in which the following elements are incorporated to the first embodiment of **Figures 2** and **3**:

- an air inlet (7) open to the atmosphere, allowing the pass of air into the intake channel,
- a secondary microfluidic channel (5) connected to the microfluidic intake channel (8) and connected to the atmosphere by the air inlet (7) allowing the entrance of air. This secondary channel (5) is placed before the measuring chamber (2), and it is connected with the intake channel (8) at a microfluidic junction (T-junction).
- a stop passive valve (6) interposed at the secondary microfluidic channel (5), and adapted to impede the fluid to flow out of the secondary microfluidic channel (5) towards the atmosphere. For that, the stop valve (6) is configured to feature a bursting pressure (BP) greater than any other pressure of the microfluidic system to avoid being surpassed, specially its bursting pressure is greater than the maximum biofluid generation pressure.

In the embodiment of **Figure 4,** the secondary channel (5) opens to the atmosphere allows the creation of air bubbles in the middle of the channel in a passive manner. The entrance of air comes from the air vent (7) when the fluid in the main intake channel (8) is drained due to the high pressure generated by the passive pump (4), creating an air bubble at the microfluidic junction of the channels (5,8). As the pump (4) is still connected to the fluid, the air bubble travels along the microfluidic main channel (8) (embodiment 1), restoring the functionality of the retention passive valve (3) when the interface arrives there. This way, a similar cycle as the one described for the embodiment of **Figures 2** and **3,** is created.

**Figure 4** additionally represents the scheme of the working principle and the different stages of the microfluidic system proposed in this second embodiment. At first **(Stage A),** fluid coming from the inlet (1) fills the measuring chamber (2) at a given flow rate, while the secondary channel (5) is also filled but up to the stop valve (6), where it is stopped. When fluid arrives at the retention passive valve (3), it is temporarily stopped **(Stage B).** When the valve (3) bursts **(Stage C),** the passive pump (4) is connected to the fluid (fluid reaches the pump) and drains it rapidly, generating an air bubble at the microfluidic junction **(Stage D).**

As the passive pump (4) is still connected to the remaining fluid, it keeps draining making the air bubble to travel along the measuring chamber (2) until the retention passive valve (3) **(Stage E).** Once there, the retention passive valve (3) has again an air-fluid interface and stops the flow as described previously for a few minutes **(Stage F).**

In **Figure 4**, $P_3$, $P_5$, $P_6$ are the fluid pressures at each location of the microfluidic circuit, whereas $P_{fluid}$ refers to the dominant pressure governing fluid flow.

The capillary pump must provide a capillary pressure greater than the maximum biofluid generation pressure. In the case of sweat applications, it has been noted that the maximum secretory pressure is below 6 kPa. Therefore, capillary pressures of the papers/capillary pumps used should be designed to be at least higher than 6 kPa. In the case of the second embodiment **(Figure 4)** the capillary pressure of the pump must be greater than inlet (in order to drain air to the junction), and below a maximum capillary pressure that avoids disconnection before air bubble travels to the retention valve.

In addition, the retention valve bursting pressure (BP) should be designed to be within the biofluid generation pressure. Preferably, the bursting pressure (BP) of the retention valve is within the range 2.4 - 6 kPa. This way, it will be able to burst eventually, but also stopping flow temporarily. The retention time will be a factor that would depend on the BP (proportionally), but subject to other system conditions. It should be remembered than once it is surpassed by the interface, the retention valve just behaves as a fluid resistor.

Furthermore, the stop valve bursting pressure should be designed to be greater than the maximum biofluid generation pressure. This way, the pressure barrier created will never be surpassed by the fluid pressure.

As it can be noted, all the above conditions are related to the biofluid generation pressure (secretory pressure for sweat glands, in the case of sweat). There is not a concrete relationship between the BP of the valves and the capillary pressure of the pump by design. They are conditioned by their relationship with the fluid generation pressure. This relationship generates the phenomenon that has been found, that is why, in **Figures 3** & **4**, flow rate was chosen to represent each state of the cycle, as it is the observable variable. However, we can define the pressures involved at each state of the cycle as described for **Figures 3** & **4**.

At first, fluid flow is directed by the biofluid pressure as it fills the measuring chamber. When fluid encounters the passive valves, the pressure at each valve is not enough to overcome the pressure barrier generated by their geometry (BP). However, as the BP of the retention (delay) valve is found within its range, eventually the liquid will burst (when $P_3$ is greater than $BP_{delay}$), and when this occurs, the fluid connects with the passive pump and its great capillary pressure dominates now the flow rate that is increased in the microfluidic circuit.

As commented previously, the pressure mismatch between pump and inlet produces a disconnection of the fluid flow at the retention valve, regenerating the air-liquid interface there and stopping the flow, as the fluid pressure now is limited to the inlet (pump is not connected to fluid). Then, the cycle starts again. In the case of the second embodiment, pump disconnection is achieved by the air arrival to the delay valve, restoring its functionality as pressure barrier.

Preferably, the system of any of the two embodiments described above, further includes a cycle detector adapted to detect when the measuring chamber is filled with biofluid, and to monitor changes on measuring cycle frequency. This cycle detector can be embodied as two electrodes arranged to measure an electric parameter at the microfluidic circuit between the inlet and the fluid pump inlet, so as to detect whether there is a biofluid or air in the microfluidic circuit.

For example, for first embodiment of **Figures 2** and **3**, the cycle detector can be located preferably just after the retention passive valve (3), and in the case of the second embodiment of **Figure 4**, it could also be located in the microfluidic junction or at the sensing chamber, as the air bubble would travel through them.

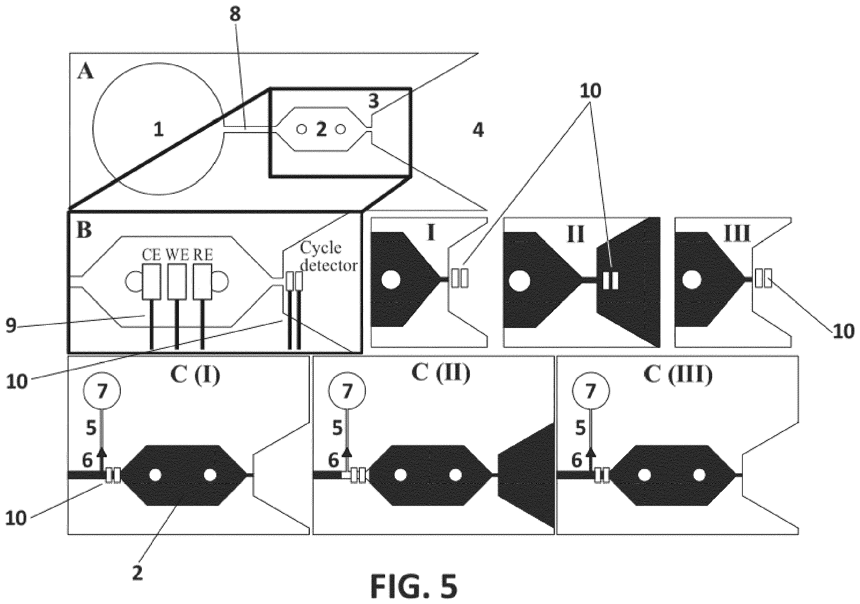

**Figure 5** shows in **Figure 5A** a practical design of the embodiment 1 **(Figures 2** & **3)**, and in **Figure 5B** it shows (as an enlarged view of the measuring chamber) several amperometric sensors (9) (CE,WE,RE) arranged inside the measuring chamber (2) for a metabolite such as lactate, and the two electrodes (10) of the cycle detector arranged just downstream the retention passive valve (3), at the zone where fluid connects to the passive pump (4). In **Figure 5B** a cycle to be detected is represented as the sequence I,II and III.

In Figure 5C a similar device as the one in **Figure 5B** is represented, but corresponding to the second embodiment **(Figures 2** & **3)**, wherein the cycle detector is adapted to detect air bubble formation, such that the two electrodes (10) are arranged between the microfluidic "T" junction and the entrance of the measuring chamber (2).

Since the cycle detector monitors the electrical resistance between the electrodes, it is possible to know at a certain moment in which cycle state is the process. A high resistance would imply that there is air in that position, meaning, in a configuration such as **Figure 5B**, that the liquid is stopped at the delay valve, and it is time to perform the amperometric measurement. In addition, cycle frequency can be calculated from the time difference between this resistance change. In another configuration, Figure 5C, a similar system could be used to detect the presence of the air bubble generated at the junction, being able to extract also the cycle state and frequency.

Given a known system, variations on cycle frequency could be attributed solely to the biofluid generation rate, as the valve and pump parameters would be designed beforehand. This way, cycle frequency could be related to biofluid generation rate, a parameter of interest in sweat applications.

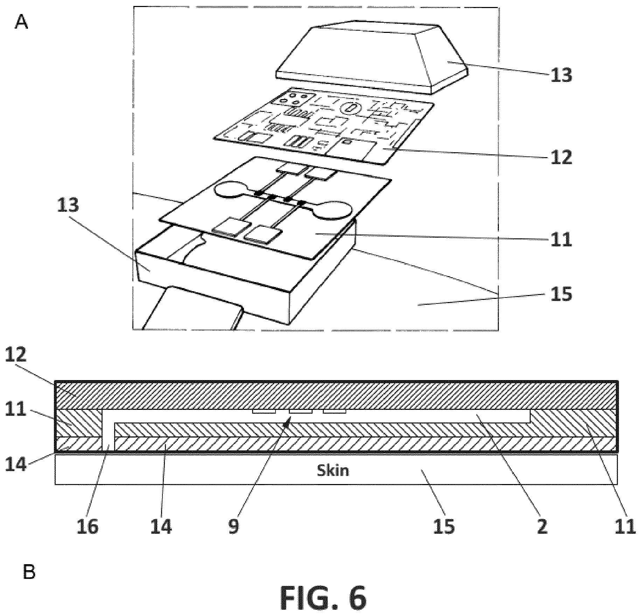

**Figure 6** shows a representation of a wereable device integrating the microfluidic system of the invention, wherein the system is configured as a disposable cartridge detachably coupled with the wearable device. In particular, the exploded view of **Figure 6A** shows two layers: a first layer (11) including the microfluidic system with sensors, and a second layer (12) on top of the first one, incuding electronics for processing data generated by the sensors, and the two layers enclosed inside a two parts casing (13).

**Figure 6B** shows an schematic representation of the two layers arrangement, wherein the bottom later (14) is an adhesive layer suitable to be adhered to the skin (15) of a subject, and having an opening (16) for sweat collection from the skin. The first layer (11) includes the microfluidic system with the structure defined above, and a second layer (12) that integrates the electronics and the sensors (9), and that together with the first layer (11) forms the measuring chamber (2).

Regarding fabrication, the microfluidic system could be constructed by assembling two or three layers. In a two-layer structure, the microfluidic channels would be fabricated on one by any convenient microfabrication procedure (soft lithography, injection moulding, ...) and closed by the second layer. In a three-layer structure, the microfluidic channels would be fabricated on the middle layer by any convenient microfabrication procedure (laser cutting, ...) and the other layers would close the microfluidic system.

Regarding fluid collection and stimulation, and more specifically for sweat dedicated applications, sweat generation could be induced by exercise or thermal action or stimulated iontophoretically either by pilocarpine or carbachol.

Regarding skin integration, the system described could add a double-sided adhesive layer (acrylic preferably) to conformally bond to skin and the microfluidic system with the convenient openings to allow collection of sweat generated. This adhesive layer could be part also of the microfluidic system as one of the layers described before.

Experimental results

For the first experiments, soft lithography was used as fabrication procedure since it provides the needed resolution to fabricate the structures needed. The material used was PDMS, a common material in the field of microfluidics, whose transparency allowed to characterize fluid behaviour by simply visualizing a coloured fluid. Paper was used as passive pump due to its simplicity and easy to change properties by simply changing the type of paper. Two different papers have been used successfully, but the cycle period changed keeping the rest of conditions constant, showing the tunability of this parameter by design. Fluid is injected continuously at the entrance of the microfluidic system at a constant flow rate (200 nL/min) which is found within the range of perspiration rates.

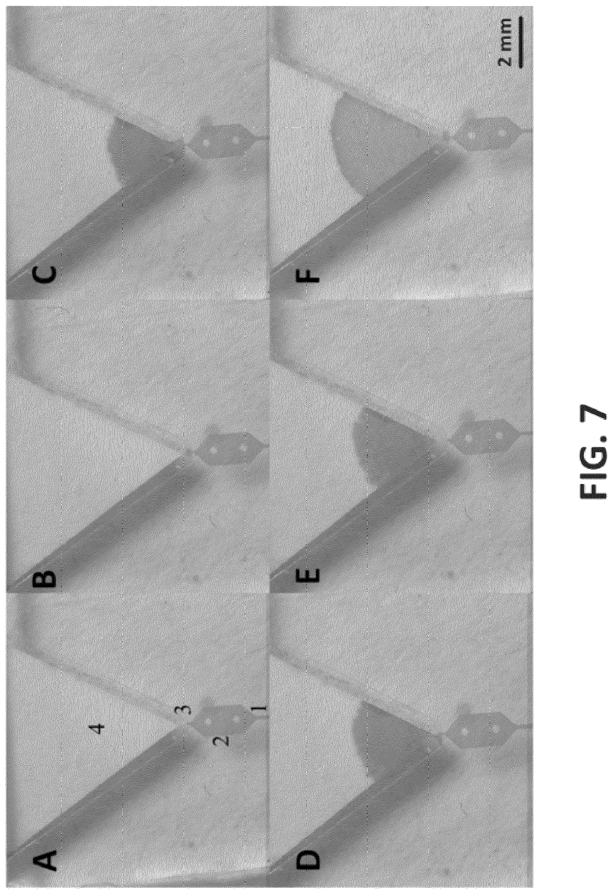

**Figure 7** captions of the different stages of the cycle of the microfluidic device in the first embodiment. Fluid arrives at the retention valve (A) and, after some minutes stopped, it bursts (B) connecting to the paper (C). After some seconds, the fluidic connection start to disappear (D) until it eventually does (E). After several minutes, the retention valve bursts again (F) repeating the whole cycle over again.

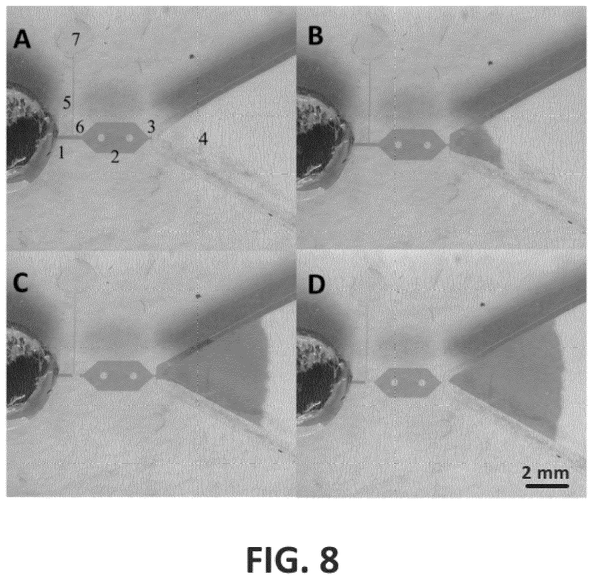

**Figure 8** shows images of different stages of a cycle of the microfluidic device in the second embodiment. Fluid arrives at the retention valve and is stopped for several minutes (A). When it bursts, it connects with the paper (B). During the connection, the fluid that has gone into the side channel returns back, due to the wicking force of the paper, creating an air bubble in the main channel (C). Then, the fluidic connection with paper disappears (D) and the air bubble remains pinned in the main channel.

Table 1 shows characteristics of the passive valve tested once fabricated. As described previously, the bursting pressure (BP) of the stop passive valve (6) should be greater than the maximum sweat secretory pressure achieved. In particular, the BP of a tested passive valve are comprehended in Table 1:

| Type of Valve | Width (um) | Betha (°) | BP (Pa) |
|---|---|---|---|
| Retention Passive Valve | 75 | 90 | 2406 |
| Stop Passive Valve | 25 | 110 | 6235 |

Table 1. Description of the different parameters used for the passive valves (both retention valve and stop valve) tested wherein BP is calculated from Equation 1, considering h=100 um, sweat surface tension of 0.072 N/m and a PDMS contact angle of 110°).

Other preferred embodiments of the present invention are described in the appended dependent claims and the multiple combinations of those claims.

**Claims**

1. Microfluidic system for continuous monitoring of metabolites and/or properties of biofluids, the system comprising:

    one measuring chamber,
    at least one inlet for the input of a biofluid,
    a microfluidic intake channel fluidly communicating the inlet with the measuring chamber,
    at least one sensor suitable for measuring a parameter of an analyte of a biofluid, and arranged to measure the parameter of a biofluid contained in the measuring chamber,
    a passive fluid pump fluidly communicated with the measuring chamber, and adapted to generate a capillary pressure greater than the biofluid generation pressure,
    a retention valve interposed between the measuring chamber and the fluid pump, wherein the retention passive valve is configured to stop flow of biofluid for a certain period of time, when the measuring chamber if filled with biofluid.

2. A system according to claim 1, further comprising:

a secondary microfluidic channel connected to the microfluidic intake channel and connected to the atmosphere, and
a stop passive valve interposed at the secondary microfluidic channel, and adapted to impede fluid flow out of the secondary microfluidic channel towards the atmosphere.

3. A system according to claim 2, wherein the stop valve is configured such that its bursting pressure is greater than the maximum biofluid generation pressure.

4. A system according to claim 2, wherein the bursting pressure of the retention passive valve is lower than the bursting pressure of the stop passive valve, and wherein preferably the bursting pressure of the retention valve is within the range 2.4 - 6 kPa.

5. A system according to any of the preceding claims, wherein the retention valve is configured to feature a bursting pressure that retain the measuring chamber filled with biofluid and stop biofluid flow for a period within the range 1 minute to 5 hours, and preferably within the range 1 to 5 minutes.

6. A system according to any of the preceding claims, wherein the retention passive valve is configured as a sudden enlargement of the cross-section area of the microfluidic intake channel.

7. A system according to any of the claims 1 to 5, wherein the retention passive valve is configured as a chemical modification of the surface of the microfluidic intake channel.

8. A system according to any of the preceding claims, wherein the fluid pump is a micro-machined capillary pump capable of forcing fluid circulation by capillary action, and wherein preferably the passive fluid pump is adapted to generate a capillary pressure higher than 6 kPa.

9. A system according to any of the claims 1 to 7, wherein the fluid pump is a porous material pump.

10. A system according to any of the preceding claims, further comprising a cycle detector adapted to detect when the measuring chamber is filled with biofluid, and to monitor changes on cycle frequency.

11. A system according to claim 10, wherein the cycle detector comprises two electrodes arranged to measure an electric parameter at the microfluidic circuit between the inlet and the fluid pump inlet, so as to detect whether there is a biofluid or air in the microfluidic circuit.

12. A system according to any of the preceding claims, wherein the inlet is adapted to collect sweat from the skin of a subject.

13. A system according to any of the preceding claims, adapted to monitor sweat metabolites and/or properties, and wherein the sweat properties include conductivity and/or sweat sweat rate and/or sweat pH, and/or ions.

14. A system according to any of the preceding claims further comprising an electronic device electrically communicated with the sensor, and adapted for processing data generated by the sensor.

15. A wearable device for sweat monitoring incorporating a system as the one defined in any of the preceding claims, and wherein the system is configured as a disposable cartridge detachably coupled with the wearable device.

**FIG. 1**
**(prior art)**

a) Filling (Q=Q$_{inlet}$)   b) Measurement (Q=0)

c) Bursting (Q=Q$_{pump}$)

d) Measurement (Q=0)   e) Bursting (Q=Q$_{pump}$)

**FIG. 2**

FIG. 3

a) Filling $(Q=Q_{inlet})$

$P_{fluid}=P_{inlet}$

b) Measurement $(Q=0)$

$P_3< BP_{delay\ valve}$
$P_6< BP_{stop\ valve}$

c) Bursting $(Q=Q_{pump})$

Whem $P_3 >BP_{delay\ valve}$
$P_{fluid}= CP_{pump}$

d) Formation of air bubble at junction $(Q=Q_{pump})$

When $P_5 >P_6$ , air drained
$P_{fluid}= CP_{pump}$

e) Air bubble travelling to valve $(Q=Q_{pump})$

When $P_3 >P_5$ , air travels
$P_{fluid}= CP_{pump}$

f) Next measurement $(Q=0)$

$P_3< BP_{delay\ valve}$
$P_6< BP_{stop\ valve}$

**FIG. 4**

EP 4 154 977 A1

FIG. 5

FIG. 6

FIG. 7

FIG. 8

## INTERNATIONAL SEARCH REPORT

| International application No |
|---|
| PCT/ES2020/070381 |

**A. CLASSIFICATION OF SUBJECT MATTER**
INV. B01L3/00    A61B5/145    A61B5/00
ADD.

According to International Patent Classification (IPC) or to both national classification and IPC

**B. FIELDS SEARCHED**

Minimum documentation searched (classification system followed by classification symbols)
B01L  A61B

Documentation searched other than minimum documentation to the extent that such documents are included in the fields searched

Electronic data base consulted during the international search (name of data base and, where practicable, search terms used)
EPO-Internal, WPI Data

**C. DOCUMENTS CONSIDERED TO BE RELEVANT**

| Category* | Citation of document, with indication, where appropriate, of the relevant passages | Relevant to claim No. |
|---|---|---|
| X<br>Y | US 2020/138347 A1 (HEIKENFELD JASON C [US]) 7 May 2020 (2020-05-07) paragraphs [0035], [0040], [0052] - [0054], [0062], [0077], [0080] - [0086]; figures 10, 11A<br>----- | 1,5,8,9, 12-15<br>6 |
| X<br>Y | WO 2018/067412 A1 (GEN ELECTRIC [US]) 12 April 2018 (2018-04-12) paragraphs [0021], [0027], [0031] - [0032], [0038] - [0040]; figures 3-5<br>----- | 1,8-10, 12-15<br>6 |
| X | US 2019/008448 A1 (BEGTRUP GAVI [US] ET AL) 10 January 2019 (2019-01-10) paragraphs [0059], [0062], [0067] - [0068], [0074]; figures 6, 7, 8A-B<br>-----<br>-/-- | 1-4,6,7, 11,13,15 |

[X] Further documents are listed in the continuation of Box C.    [X] See patent family annex.

* Special categories of cited documents :
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 8 February 2021 | 17/02/2021 |

| Name and mailing address of the ISA/ European Patent Office, P.B. 5818 Patentlaan 2 NL - 2280 HV Rijswijk Tel. (+31-70) 340-2040, Fax: (+31-70) 340-3016 | Authorized officer Ruiz-Echarri Rueda |

Form PCT/ISA/210 (second sheet) (April 2005)

## INTERNATIONAL SEARCH REPORT

International application No

PCT/ES2020/070381

C(Continuation).    DOCUMENTS CONSIDERED TO BE RELEVANT

| Category* | Citation of document, with indication, where appropriate, of the relevant passages | Relevant to claim No. |
|---|---|---|
| Y | WO 2014/058750 A1 (GEN ELECTRIC [US]) 17 April 2014 (2014-04-17) | 6 |
| A | paragraphs [0068], [0078], [0101]; figures 1-3 | 1 |
| | ----- | |
| A | WO 2019/075573 A1 (THE ROYAL INSTITUTION FOR THE ADVANCEMENT OF LEARNING/MCGILL UNIV [CA]) 25 April 2019 (2019-04-25) paragraphs [0018], [0021], [0033], [0041] - [0048], [0069] - [0069], [0087], [0090]; claims 1-8; figures 1a-c | 1-14 |
| | ----- | |

## INTERNATIONAL SEARCH REPORT
Information on patent family members

| International application No |
|---|
| PCT/ES2020/070381 |

| Patent document cited in search report | | Publication date | Patent family member(s) | | Publication date |
|---|---|---|---|---|---|
| US 2020138347 | A1 | 07-05-2020 | NONE | | |
| WO 2018067412 | A1 | 12-04-2018 | NONE | | |
| US 2019008448 | A1 | 10-01-2019 | NONE | | |
| WO 2014058750 | A1 | 17-04-2014 | CA | 2886462 A1 | 17-04-2014 |
| | | | CA | 2886469 A1 | 17-04-2014 |
| | | | CA | 2886470 A1 | 17-04-2014 |
| | | | CA | 2886777 A1 | 17-04-2014 |
| | | | CN | 104704369 A | 10-06-2015 |
| | | | CN | 104704370 A | 10-06-2015 |
| | | | CN | 104838267 A | 12-08-2015 |
| | | | CN | 104838268 A | 12-08-2015 |
| | | | EP | 2904399 A2 | 12-08-2015 |
| | | | EP | 2904400 A1 | 12-08-2015 |
| | | | EP | 2904401 A2 | 12-08-2015 |
| | | | EP | 2904402 A1 | 12-08-2015 |
| | | | EP | 3425401 A1 | 09-01-2019 |
| | | | JP | 6319590 B2 | 09-05-2018 |
| | | | JP | 6322847 B2 | 16-05-2018 |
| | | | JP | 6426611 B2 | 21-11-2018 |
| | | | JP | 6479663 B2 | 06-03-2019 |
| | | | JP | 2015530601 A | 15-10-2015 |
| | | | JP | 2015531488 A | 02-11-2015 |
| | | | JP | 2015531489 A | 02-11-2015 |
| | | | JP | 2015533218 A | 19-11-2015 |
| | | | US | 2015233916 A1 | 20-08-2015 |
| | | | US | 2015233917 A1 | 20-08-2015 |
| | | | US | 2015260719 A1 | 17-09-2015 |
| | | | US | 2015293097 A1 | 15-10-2015 |
| | | | US | 2018017562 A1 | 18-01-2018 |
| | | | US | 2018156795 A1 | 07-06-2018 |
| | | | US | 2019018013 A1 | 17-01-2019 |
| | | | US | 2020049707 A1 | 13-02-2020 |
| | | | WO | 2014058750 A1 | 17-04-2014 |
| | | | WO | 2014058757 A2 | 17-04-2014 |
| | | | WO | 2014058758 A1 | 17-04-2014 |
| | | | WO | 2014058760 A2 | 17-04-2014 |
| WO 2019075573 | A1 | 25-04-2019 | US | 2020346211 A1 | 05-11-2020 |
| | | | WO | 2019075573 A1 | 25-04-2019 |

Form PCT/ISA/210 (patent family annex) (April 2005)